# EUROPÄISCHE PATENTSCHRIFT

(11) **EP 3 244 934 B1**
(45) Veröffentlichungstag und Bekanntmachung des Hinweises auf die Patenterteilung: **09.05.2018**
(21) Anmeldenummer: 16823167.8
(22) Anmeldetag: 08.12.2016
(51) Int. Cl.: A61L 2/22, G21F 7/04

(54) **DEKONTAMINATIONSANORDNUNG**
DECONTAMINATION ASSEMBLY
SYSTÈME DE DÉCONTAMINATION

(30) Priorität: 07.04.2016 DE 102016106407
(43) Veröffentlichungstag der Anmeldung: 22.11.2017
(73) Patentinhaber: Metall + Plastic GmbH, 78315 Radolfzell (DE)
(72) Erfinder: KLEINMANN, Stefan, 78315 Radolfzell (DE)
(74) Vertreter: Patentanwälte Behrmann Wagner PartG mbB
(86) Internationale Anmeldenummer: PCT/EP2016/080337
(87) Internationale Veröffentlichungsnummer: WO 2017/174169

(56) Entgegenhaltungen:
- WO-A1-2011/085735
- WO-A1-2013/003967
- WO-A1-2014/079779
- US-A- 6 010 400

## Beschreibung

Die Erfindung betrifft eine Dekontaminationsanordnung gemäß dem Oberbegriffs des Anspruchs 1, insbesondere für pharmatechnische Anwendungen, umfassend einen Isolator mit einem Manipulatorraum (auch als Arbeitskammer bzw. -raum bezeichnet) und einem (entlang einer Vertikalen) oberhalb des Manipulatorraums angeordneten Plenumraum (Zwischenraum), der über mindestens ein in einem Umlufterzeugerraum angeordnetes Umluftgebläse mit einem Luftstrom versorgbar ist, wobei zwischen Plenumraum und Manipulatorraum eine Membran, insbesondere ein Gewebe, zum Erzeugen einer laminaren Luftströmung im Manipulatorraum (aus dem in den Plenumraum einströmenden Luftstrom) während eines Arbeitsbetriebszustandes angeordnet ist, wobei der Manipulatorraum luftleitend mit dem Umlufterzeugerraum über einen Rückluftkanal verbunden ist, der bevorzugt zwischen zwei durchsichtigen Scheiben angeordnet ist, weiter umfassend Feinstverteilmittel, insbesondere einen Verdampfer oder Zerstäuber, zum Erzeugen eines Dekontaminationsmittel-/Luftgemisches auf Basis eines flüssigen Dekontaminationsmittelvorrats, vorzugsweise eines H₂O₂-Vorrats, wobei die Feinstverteilmittel über mindestens eine in den Plenumraum einmündende Plenum-Versorgungsleitung mit dem Plenumraum verbunden sind, um den Plenumraum unmittelbar mit dem Dekontaminationsmittel-/Luftgemisch in einem Dekontaminationsbetriebszustand zu versorgen.

Ferner betrifft die Erfindung ein Verfahren zum Betreiben einer solchen Dekontaminationsanordnung gemäß dem Oberbegriff des Anspruchs 14.

In der WO 2011/085735 A1 bzw. EP 2 719 962 A1 ist eine Dekontaminationsanordnung, umfassend einen Inkubator sowie einen H₂O₂-Verdampfer beschrieben. Der H₂O₂-Verdampfer(Blitzverdampfer) ist über eine Plenum-Versorgungsleistung unmittelbar mit einem in der Druckschrift als Zwischenraum bezeichneten Plenumraum verbunden, der von einem darunter befindlichen Manipulatorraum über eine Membran zur Erzeugung einer laminaren Luftströmung getrennt ist. In einer Dekontaminationsphase wird über die Plenum-Versorgungsleitungen ein Dekontaminationsmittel-/Luftgemisch in den Plenumraum eingeleitet. Gleichzeitig ist eine Abluftleitung aus einem oberhalb des Plenumraums befindlichen Umlufterzeugerraums geöffnet, so dass das Dekontaminationsmittel-/Luftgemisch langsam durch die Membran in den Manipulatorraum und über seitliche Rückluftkanäle in den Umlufterzeugerraum wandert. Nachteilig bei dem bekannten Verfahren ist die vergleichsweise lange Dekontaminationszeit, die benötigt wird, um eine ausreichende Kontaktzeit, insbesondere des unteren Bereichs des Manipulatorraums, mit dem Dekontaminationsmittel-Luftgemisch zu ermöglichen.

Aus der WO 2013/003967 A1 sowie der hierzu prioritätsbegründenden CH 705 249 A1 sind verschiedene Ausführungsvarianten von Dekontaminationsvorrichtungen, umfassend einen H₂O₂-Zerstäuber sowie einen Inkubator bekannt. Eine erste beschriebene Möglichkeit zur Platzierung des Zerstäubers besteht darin, den Zerstäuber im Manipulatorraum (dort als Arbeitskammer bezeichnet) anzuordnen. Alternativ ist es möglich, den Zerstäuber außerhalb der Arbeitskammer, benachbart zu dieser anzuordnen und das Dekontaminationsmittel-/Luftgemisch unmittelbar in die Arbeitskammer einzuleiten. Eine weitere beschriebene Alternative sieht vor, den Zerstäuber unmittelbar im Umlufterzeugerraum anzuordnen. Zum Verteilen des Dekontaminationsmittel-/Luftgemisches wird das Umluftgebläse betrieben.

Aus der US 6,010,400 A ist es bekannt, Luft im Umluftbetrieb durch einen Verdampfer zu fördern, d.h. diesen nicht mit steriler Druckluft von außen zu versorgen, wobei das resultierende Dekontaminations-/Luftgemisch unmittelbar in einen Manipulatorraum eingeleitet wird, unter Umgehung eines katalysatorgetränkten Hochleistungsschwebstofffilter in einem Bereich oberhalb des Manipulatorraums.

Bei der bekannten Dekontaminationsanordnung befindet sich das Umluftgebläse nicht in einem Umlufterzeugerraum in einem Bereich oberhalb des Manipulatorraums sondern ist seitlich in einem Rohrleitungssystem angeordnet.

Ausgehend von dem vorgenannten Stand der Technik liegt der Erfindung die Aufgabe zugrunde eine Dekontaminationsanordnung mit einem oberhalb eines Manipulatorraums angeordneten Plenumraum anzugeben, der über eine Membran zur Erzeugung einer laminaren Strömung im Manipulatorraum (Arbeitsraum) vom Manipulatorraum getrennt ist, bei der die Dekontaminationszeit, d.h. die Beaufschlagungszeit mit einem Dekontaminationsmittel/Luftgemisch gekürzt ist.

Ferner besteht die Aufgabe darin, ein Verfahren zum Betreiben einer solchen Dekontaminationsanordnung anzugeben, wobei sich das Verfahren durch einen verkürzten Dekontaminationsbetriebszustand auszeichnen soll.

Diese Aufgabe wird hinsichtlich der Dekontaminationsanordnung mit den Merkmalen des Anspruchs 1 gelöst, d.h. bei einer gattungsgemäßen Dekontaminationsanordnung dadurch, dass die Feinstverteilmittel über mindestens eine Manipulator-Versorgungsleitung unmittelbar mit dem Manipulatorraum verbunden sind, um den Manipulatorraum in den Dekontaminationsbetriebszustand unmittelbar mit dem Dekontaminationsmittel-/Luftgemisch zu versorgen.

Hinsichtlich des Verfahrens wird die Aufgabe mit den Merkmalen des Anspruchs 14 gelöst, d.h. bei einem gattungsgemäßen Verfahren, bei dem der Plenumraum unmittelbar mit dem Dekontaminationsmittel-/Luftgemisch beaufschlagt wird, dadurch, dass zusätzlich der Manipulatorraum unmittelbar mit den Dekontaminationsmittel-/Luftgemisch versorgt wird und zwar über mindestens eine unmittelbar in den Manipulatorraum einmündende Manipulator-Versorgungsleitung, die die Feinstverteilmittel unmittelbar mit dem Manipulatorraum verbindet.

Vorteilhafte Weiterbildungen sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen. Zur Vermeidung von Wiederholungen sollen vorrichtungsgemäß offenbarte Merkmale auch als verfahrensgemäß offenbart gelten und beanspruchbar sein. Ebenso sollen verfahrensgemäß offenbarte Merkmale auch als vorrichtungsgemäß offenbart gelten und beanspruchbar sein.

Der Erfindung liegt der Gedanke zugrunde, die Feinstverteilmittel sowohl über eine unmittelbar in den Plenumraum oberhalb der Membran einmündende bzw. dort einen Auslass aufweisende Plenum-Versorgungsleitung (Direktleitung) mit dem Plenumraum zu verbinden als auch über mindestens eine Manipulator-Versorgungsleitung (Direktleitung) unmittelbar mit dem Manipulatorraum. Die Plenumversorgungsleitung mündet unmittelbar in den Plenumraum, d.h. bevorzugt nicht in eine etwaige (sonstige) Zu- oder Ableitung in den bzw. aus dem Plenumraum. Anlaog mündet die Manipulator-Versorgungsleitung unmittelbar in den Manipulatorraum, d.h. nicht in eine Zu- oder Ableitung in den bzw. aus dem Manipulatorraum (Arbeitsraum). Selbstverständlich können bei Bedarf derartige zusätzliche Zu- oder Ableitungen vorgesehen werden. Kerngedanke der Erfindung ist es jedoch, die Feinstverteilmittel direkt bzw. unmittelbar sowohl mit dem Plenumraum als auch mit dem Manipulatorraum zu verbinden, so dass sowohl der Plenumraum als auch der Manipulatorraum, bevorzugt gleichzeitig mit einem Dekontaminationsmittel-/Luftgemisch von den Feinstverteilmitteln versorgbar sind bzw. beaufschlagt werden.

Kerngedanke der Erfindung ist es also das Dekontaminationsmittel-/Luftgemisch nicht nur in einen einzigen Funktionsraum des Inkubators (Isolators) der Dekontaminationsanordnung einzublasen und dann aus diesem Raum in die anderen Räume zu verteilen, sondern unmittelbar in die zwei über die Laminarisierungsmembran getrennten Räume nämlich den, bevorzugt unterhalb des Umluftgebläses befindlichen, Plenumraum und den entlang der Vertikalen darunter befindlichen Manipulatorraum einzublasen, um somit eine schnellere Verteilung des Dekontaminationsmittel-/Luftgemisches zu erreichen und damit die Dekontaminationsphase deutlich zu verkürzen.

Wesentlicher Vorteil der erfindungsgemäßen Dekontaminationsanordnung bzw. des erfindungsgemäßen Betriebsverfahrens für eine solche Anordnung besteht darin, dass aufgrund der unmittelbaren Anbindung sowohl des Plenumraums als auch des Manipulatorraums an die Feinstverteilmittel eine schnelle Kontaktierung sämtlicher relevanten Bereiche mit dem Dekontaminationsmittel-/Luftgemisch erreicht werden kann, ohne dass es hierzu notwendig ist, das Umluftgebläse anzuschalten. Dieses ist daher bevorzugt während der Dekontaminationsphase (Zeit ab dem Beginn des Einlasses des Dekontaminationsmittel-/Luftgemisches bis zum Beginn der Freispülphase), zumindest während des größten Teils bzw. Zeitabschnitts der Dekontaminationsphase ausgeschaltet und die Verteilung des Dekontaminationsmittel-/Luftgemisches im Isolator (= Inkubator) wird lediglich erreicht, durch die Einspeisung des Dekontaminationsmittel-/Luftgemisches sowohl im Plenumraum als auch im Manipulatorraum mit Hilfe von externer Trägerluft und die Öffnung eines Abluftkanals aus dem Isolator, über den das über die Feinstverteilmittel zugeführte Trägerluftvolumen entweder unmittelbar oder über einen Katalysator zum Abbau von Dekontaminationsmitteln nach außen aus dem Isolator abgeleitet werden kann. Diese Ausführungsform ermöglicht dann eine später noch zu erläuternde, bevorzugte Ausgestaltung bzw. Weiterbildung der Dekontaminationsanordnung, bei der dem mindestens einen Umluftgebläse mindestens ein Katalysator zum Abbau von Dekontaminationsmittel während einer auf die Dekontaminationsphase bzw. dem Dekontaminationsbetriebszustand folgende Freispülphase Dekontaminationsmittel im Umluftbetrieb abgebaut werden kann. Würde bei einer solchen Anordnung nämlich das Umluftgebläse ohne das Vorsehen eines Bypasses zur Verteilung des Dekontaminationsmittel-/Luftgemisches während der Dekontaminationsphase betrieben, würde dies zu einem unerwünschten Abbau des Dekontaminationsmittels bereits während der Dekontaminationsphase führen.

Ein weiterer Vorteil des Nicht-Betreibens des Umluftgebläses zumindest während des größten Zeitabschnitts während der Dekontaminationsphase besteht darin, dass ein vorzugsweise das Umluftgebläse von dem Plenumraum trennender Hochleistungsschwebstofffilter während der Dekontaminationsphase im Wesentlichen nur von unten, d.h. an der Kontaktfläche zum Plenumraum mit dem Dekontaminationsmittel-/ Luftgemisch in Berührung kommt und nicht durchspült wird - ein solches Durchspülen bringt nämlich den Nachteil einer verlängerten Freispulzeit mit sich, da der Hochleistungsschwebstofffilter Dekontaminationsmittel aufnimmt, welches dann noch längere Zeit aus dem Hochleistungsschwebstofffilter, insbesondere einen HEPA- oder ULPA-Filter ausdampft.

Der Erfinder hat erkannt, dass bei der bisherigen Anordnung von H₂O₂-Dampfauslässen oberhalb der Membran aufgrund der Membran im Plenumraum noch turbulente Strömungen laminarisiert wurden (was auch die Aufgabe der Membran im Produktionsbetrieb ist) und dadurch lange Verteilzeiten im Manipulatorraum resultiert haben. Durch die erfindungsgemäße Ausleitung von H₂O₂ sowohl im Plenumraum als auch im Manipulatorraum können in beiden Räumen, insbesondere durch Ausleitung jeweils an mehreren unterschiedlichen über den jeweiligen Raum verteilten Stellen unmittelbar turbulente Strömungsverhältnisse erzeugt werden, wozu es bevorzugt ist, wie später noch erläutert werden wird, hohe Austrittsgeschwindigkeiten von H₂O₂-haltiger Druckluft (Trägerluft) im Plenumraum und im Manipulatorraum zu realisieren. Durch die Erzeugung turbulenter Strömungsverhältnisse unmittelbar durch das ausströmende H₂O₂-haltige Druckluftmedium kann zudem auf sogenannte Turbulatoren in Form von kleinen unmittelbar im Manipulatorraum angeordneten (Verwirbelungs-) Lüftern verzichtet werden, wodurch wiederum ein Partikeleintrag durch rotierende mechanische Bauteile vermieden werden kann.

Vergleichsversuche der Anmelderin haben ergeben, dass die Zykluszeit mit einer erfindungsgemäßen Vorrichtung bzw. einem erfindungsgemäßen Verfahren um mehr als 50 % reduziert werden kann und der Wasserstoffperoxidverbrauch zur Erzielung der gleichen Wasserstoffperoxidkonzentration ebenfalls um mehr als 50 %, konkret in Vergleichsversuchen gemessen 43 %.

Zusammenfassend geht die Erfindung erstmals den Weg zur unmittelbaren Beaufschlagung sowohl des Plenumraums als auch den Manipulatorraums über jeweils mindestens eine Direktleitung bzw. Versorgungsleitung mit dem Dekontaminationsmittel-/Luftgemisch, insbesondere einem H₂O₂-/Luftgemisch, wobei die jeweilige Versorgungsleitung unmittelbar in dem entsprechenden Raum, d.h. dem Plenumraum oder dem Manipulatorraum, bevorzugt über eine später noch zu erläuternde Düse ausmündet.

An dieser Stelle sei angemerkt, dass der Fachmann auch bei einer Kombination der eingangs genannten Druckschriften WO 2011/085735 A1 und der WO 2013/003967 A1 keine Veranlassung zur Verbindung der Feinstverteilmittel sowohl mit dem Plenumraum als auch mit dem Manipulatorraum hatte. So behandelt die erstgenannte Druckschrift ausschließlich die Einleitung von Wasserstoffperoxid in den Plenumraum, was aus damaliger Sicht völlig ausreichend erschien, da sich der H₂O₂-Dampf von oben nach unten durch die Membran in den Manipulatorraum verteilt hat. Die WO 2013/003967 A1 setzt dagegen keinen dezentralen H₂O₂-Verdampfer ein sondern einen Zerstäuber, durch dessen Einsatz Wasserstoffperoxid nicht in die Gasphase übergeht sondern lediglich in feinste Tröpfchen verteilt wird. Solche Zerstäuber werden nicht mit Verdampfern in einer gemeinsamen Anlage kombiniert - es handelt sich um unterschiedliche Feinstverteilkonzepte. Der Zerstäuber wird gemäß der Lehre der Druckschrift WO 2013/003967 A1 (vgl. Merkmal d)) des Anspruchs 1 als Ganzes als integraler Bestandteil der bekannten Dekontaminationsvorrichtung am oder im sogenannten Containment installiert, wobei unterschiedliche Anbringungsorte, beispielsweise alternativ im Plenumraum oder im Manipulatorraum beschrieben sind. Bereits aufgrund der Beschreibung der Zerstäubereinheit als untrennbare Einheit, die den H₂O₂-Nebel jeweils nur an einer einzigen Stelle erzeugt, hatte der Fachmann keine Veranlassung eine Aufteilung bzw. Verteilung zwischen zwei unterschiedlichen Räumen vorzusehen. Erst recht hat der Fachmann keine Veranlassung, was in Weiterbildung der Erfindung vorgesehen und im Folgenden noch beschrieben wird, den Feinstverteilmittel an mehreren unterschiedlichen Stellen im Manipulatorraum Auslässe bzw. Ausströmöffnungen, insbesondere Düsen zuzuordnen, um somit eine Ausleitung von H₂O₂ unmittelbar an oder der Nähe von kritischen Bereichen zuzuordnen.

Wie erwähnt, handelt es sich bei dem Manipulatorraum um einen Arbeitsraum, in welchem die eigentlich, bevorzugt pharmatechnische Anwendung vollzogen wird. Bevorzugt befindet sich in dem Manipulatorraum eine pharmatechnische Produktionsanlage zur zumindest teilweisen Herstellung eines Medikamentes bzw. medizinischen Wirkstoffs und/oder zur Dosierung- und/oder Portionierung eines Medikamentes bzw. medizinischen Wirkstoffs und/oder zur Abpackung eines Medikamentes bzw. medizinischen Wirkstoffs.

Der entlang einer Vertikalen oberhalb des Manipulatorraums befindliche Plenumraum dient im Wesentlichen zur Luftverteilung eines über mindestens ein in einem bevorzugt entlang der Vertikalen oberhalb des Plenumraums angeordneten Umlufterzeugerraum angeordneten Umluftgebläses zugeführten Luftstroms. Dieser strömt dann über die Membran aus dem Plenumraum in den Manipulatorraum, wobei die Membran einen, bevorzugt vertikalen, laminaren Luftstrom erzeugt, weiter bevorzugt mit einer Luftgeschwindigkeit von 0,45 m/s. In den Manipulatorraum strömt die laminare Luftströmung dann von oben nach unten und in einen unteren Bereich seitlich hin zu mindestens einem Rückluftkanal der, bevorzugt zwischen zwei durchsichtigen, bevorzugt vertikalen, Scheiben angeordnet ist, so dass die Bedienperson von außen durch den Rückluftkanal nach innen in den Manipulatorraum einsehen kann. Bevorzugt, jedoch nicht zwingend ist dem Manipulatorraum mindestens ein Port zugeordnet, der bevorzugt die den Rückluftkanal begrenzenden Scheiben durchsetzt, wobei an dem Port ein Handschuh angeordnet ist, über den die Bedienperson manuell in den Manipulator- bzw. Arbeitsraum von außen eingreifen kann. Der Rückluftkanal wiederum mündet bevorzugt in den bevorzugt entlang der Vertikalen oberhalb des Plenumraums angeordneten Umlufterzeugerraum in dem sich das mindestens eine Umluftgebläse befindet, dem bevorzugt jedoch nicht zwingend ein Katalysator zum Abbau von Dekontaminationsmittel während einer Freispülphase nach dem Dekontaminationsbetriebszustand dient. Bevorzugt ist der Umlufterzeugerraum wiederum über mindestens einen Hochleistungsstickstofffilter von dem Plenumraum getrennt. Der Isolator umfasst zusätzlich mindestens eine Abluftleitung, die bevorzugt aus dem Umlufterzeugerraum ausmündet, wobei über die Abluftleitung bevorzugt, wie erwähnt das während der Dekontaminationsphase zumindest während des Zuführens von des Dekontaminationsmittel/- Luftgemisches geöffnet ist um das zugeführte Trägerluftvolumen wieder abführen zu können. Ferner umfasst der Isolator bevorzugt mindestens eine Zuluftleitung über die sterile, konditionierte, insbesondere temperierte und/oder getrocknete sterile Frischluft zugeführt werden kann.

Als besonders vorteilhaft hat es sich herausgestellt, wenn mehrere an unterschiedlichen Stellen in den Plenumraum ausmündende Versorgungsleitungen vorgesehen sind, über den der Plenumraum mit dem Dekontaminationsmittel-/Luftgemisch versorgbar ist bzw. versorgt wird. Bevorzugt sind die Auslässe der Versorgungsleitungen gleichmäßig über die Flächenerstreckung der Membran verteilt angeordnet, um somit eine gute Verteilung zu ermöglichen. Grundsätzlich ist es möglich sämtliche Versorgungsleitungen unmittelbar, d.h. jeweils bis zu den Feinstverteilmitteln zu führen - bevorzugt ist jedoch eine Ausführungsvariante, bei der die Plenumversorgungsleitungen von einem Verteilerabschnitt einer Hauptleitung abzweigen, welche wiederum unmittelbar zu den Feinstverteilmitteln führt. Zusätzlich oder alternativ ist es bevorzugt, wenn nicht nur eine einzige, sondern mehrere Manipulatorversorgungsleitungen in den Manipulatorraum ausmünden um auch hier eine optimale Verteilung des Dekontaminationsmittel-/Luftgemisches zu erreichen. Auch hier ist es denkbar, sämtliche Manipulatorversorgungsleitungen unmittelbar bis zu den Feinstverteilmitteln zu führen, wobei es bevorzugt ist, wenn die Manipulatorversorgungsleitungen aus einer Hauptleitung bzw. einem Verteilungsleitungsabschnitt ausmünden, welcher unmittelbar an die Feinstverteilmittel angeschlossen ist. Im Hinblick auf die Anordnung der mindestens einen, bevorzugt der mehreren Manipulatorversorgungsleitungen hat es sich als vorteilhaft herausgestellt, wenn diese im Bereich der seitlichen, sich in vertikaler Richtung erstreckenden Inkubator- (Isolator-) bzw. Manipulatorraumwände angeordnet sind und das Dekontaminationsmittel-/Luftgemisch von seitlich in das Manipulatorrauminnere ausblasen. Bevorzugt befindet sich unterhalb der Auslässe der Manipulatorversorgungsleitung keine Arbeits- bzw. Funktionseinheit, so dass eine Partikelkontamination sicher vermieden wird.

Ganz besonders bevorzugt ist eine Ausgestaltung der Dekontaminationsanordnung, bei der der Plenumraum und der Manipulatorraum gleichzeitig über die mindestens eine Plenumversorgungsleitung und die mindestens eine Manipulatorversorgungsleitung unmittelbar mit dem Dekontaminationsmittel-/Luftgemisch versorgbar sind bzw. versorgt werden, zumindest während eines Zeitabschnitts der Dekontaminationsphase. Zusätzlich oder alternativ ist es während eines Zeitabschnitts der Dekontaminationsphase bei entsprechender Ausgestaltung der Dekontaminationsanordnung möglich Plenumraum und Manipulatorraum getaktet bzw. alternierend mit Dekontaminationsmittel-/Luftgemisch zu versorgen.

Während des Arbeitsbetriebszustandes, der auf einen Freispülbetriebszustand folgt und während dessen, bevorzugt pharmatechnische, Funktions- oder Arbeitseinheiten im Manipulatorraum betrieben werden, herrscht im Plenumraum verglichen mit dem Manipulatorraum ein Überdruck bzw. stellt sich ein aufgrund der für einen Strömungswiderstand sorgenden Membran zur Laminarisierung der Luftströmung. Um zu verhindern, dass aufgrund dieses Überdrucks aus dem Plenumraum Luft rückwärts über die Plenumversorgungsleitung und die Manipulatorraumversorgungsleitung in den Manipulatorraum strömt und dadurch die laminare, bevorzugt vertikale Luftströmung durch die Membran stört, ist in Weiterbildung der Erfindung vorgesehen, dass eine solche Störluftströmung verhindert wird. Das kann für den bevorzugten Fall, dass die Plenumversorgungsleitung und die Manipulatorversorgungsleitung von einem gemeinsamen Versorgungsleitungsabschnitt versorgt werden bzw. die Plenumversorgungsleitung und die Manipulatorversorgungsleitung einen gemeinsamen Leitungsabschnitt aufweisen durch entsprechend ausgebildete und angeordnete Ventilmittel verhindert werden, die von Steuermittel und/oder manuell derart betätigbar bzw. ansteuerbar sind, dass diese die Verbindung zwischen Plenumversorgungsleitung und Manipulatorversorgungsleitung unterbrechen während des Arbeitsbetriebszustandes, wobei die Verbindung vorzugsweise zumindest zeitweise während der Dekontaminationsphase zur Gewährleistung einer zeitgleichen Beaufschlagung des Plenumraums und des Manipulatorraums mit Dekontaminationsmittel-/Luftgemisch geöffnet ist bzw. wird. Alternativ ist es möglich separate Verdampfer oder Zerstäuber für den Manipulator zur Versorgung der Manipulatorversorgungsleitung und der Plenumversorgungsleitung vorzusehen, d.h. auf eine unmittelbare luftleitende Verbindung zwischen der Plenumversorgungsleitung und der Manipulatorversorgungsleitung zu verzichten.

Insbesondere um die vorerwähnte Störluftströmung zu verhindern und/oder um einen getakteten Betrieb bzw. eine getaktete bzw. alternierende Versorgung des Plenumraums und des Manipulatorraums mit dem Dekontaminationsmittel-/Luftgemisch zu ermöglichen und/oder um das Volumenstromverhältnis des durch die mindestens eine Plenumraumversorgungsleitung strömenden Dekontaminationsmittel-/Luftvolumenstroms und des durch die mindestens eine Manipulatorversorgungsleitung strömenden Dekontaminationsmittel-/Luftvolumenstroms zu variieren. Insbesondere während der Dekontaminationsphase ist in Weiterbildung der Erfindung der mindestens einen Plenumversorgungsleitung, bevorzugt vor der Aufteilung auf mehrere Versorgungsleitungen, insbesondere in einem Verteilerbereich ein Plenumventil zugeordnet und/oder der mindestens einen Manipulatorversorgungsleitung, vorzugsweise in einem Bereich vor der Verteilung auf mehrere Manipulatorversorgungsleitungen ein Manipulatorventil. Das Plenumventil und/oder das Manipulatorventil sind/ist über eine Steuereinheit und/oder manuell entsprechend der vorgenannten mindestens einen Zielsetzung betätigbar.

Wie erwähnt ist es besonders bevorzugt, wenn der Umlufterzeugerraum von dem Plenumraum über mindestens einen Hochleistungsschwebstofffilter getrennt ist, über den das mindestens eine Umluftgebläse dem Plenumraum mit dem Luftstrom versorgt. Für den bevorzugten Fall, dass das Umluftgebläse während des größten Zeitabschnitts der Dekontaminationsphase (Dekontaminationsbetriebs-zustand) ausgeschaltet ist wird verhindert, dass das Hochleistungsschwebstofffilter stark mit Dekontaminationsmitteln "aufgeladen" bzw. belastet wird. Grundsätzlich ist es nämlich für eine ausreichende Sterilität der Dekontaminationsanordnung ausreichend, wenn lediglich die Kontaktseite des Hochleistungsschwebstofffilters, d.h. die Unterseite zum Plenumraum dekontaminiert wird.

Besonders zweckmäßig ist es, wenn dem über Steuermittel ansteuerbaren Umluftgebläse mindestens ein Katalysator zum Abbau von Dekontaminationsmittel im Umluftbetrieb während einer auf die Dekontaminationsphase folgenden Freispülphase zugeordnet ist. Für den bevorzugten Fall der stationären Anordnung des Katalysators relativ zu dem Umluftgebläse ohne das Vorsehens einer Bypass-Möglichkeit bleibt das Umluftgebläse bevorzugt zumindest während des größten Zeitabschnitts der Dekontaminationsphase ausgeschaltet, um während der Dekontaminationsphase einen Abbau von Dekontaminationsmittel im Katalysator zu verhindern. Ein Betreiben des Umluftgebläses während der Dekontaminationsphase, insbesondere während des größten Teils der Dekontaminationsphase zur weiteren Optimierung der Verteilung des Dekontaminationsmittel-/Luftgemisches im gesamten Katalysator ist dann möglich bzw. in Weiterbildung der Erfindung vorgesehen, wenn zwischen dem Katalysator und dem Umluftgebläse, bevorzugt durch aktives Verstellen des Katalysators oder von diesem zugeordneten Luftleitmitteln ein Bypass erzeugbar ist, der ein Durchströmen des Katalysators während eines Umluftgebläsebetriebes in der Dekontaminationsphase verhindert. Dieser Bypass ist während der Freispülphase zum Abbau des Dekontaminationsmittels bevorzugt geschlossen.

Wie eingangs bereits angedeutet mündet die mindestens eine Plenumversorgungsleitung und/oder die mindestens eine Manipulatorversorgungsleitung über jeweils mindestens eine (Austritts-)Düse in den Plenumraum bzw. den Manipulatorraum, wobei die jeweilige Düse mehrere in unterschiedliche Richtungen weisende Austrittsöffnungen aufweist. Besonders bevorzugt ist eine Ausführungsform, bei der mehrere Plenumversorgungsleitungen über jeweils mindestens eine (Austritts-)Düse in den Plenumraum und/oder mehrere Manipulatorversorgungsleitungen über jeweils mindestens eine (Austritts-)Düse in den Manipulatorraum ausmünden, d.h., dass mehrere (Austritts-)Düsen im Plenumraum und/oder im Manipulatorraum vorgesehen sind. Besonders gute Erfahrungen wurden mit einer Düsenausführungsvariante gemacht, die einen, insbesondere endseitigen, Konusabschnitt aufweist und einen dazu benachbarten, insbesondere rückwärtigen, Zylinderabschnitt, wobei sowohl Austrittsöffnungen im Konusabschnitt als auch im Zylinderabschnitt vorgesehen sind.

Im Hinblick auf die Anordnung der Ausläse, insbesondere Austrittsdüsen für H₂O₂-haltige Trägerluft (Druckluft) hat es sich hinsichtlich des Manipulatorraums als vorteilhaft erwiesen, solche Auslässe, insbesondere Düsen im Bereich von Manipulatorraumecken anzuordnen, wobei unter einer Manipulatorecke Innenecken zwischen zwei den Manipulatorraum begrenzenden Außenwänden verstanden werden, die vorzugsweise in einem Winkel von 90° angeordnet sind. Bevorzugt wird in einer Ecke ein Auslass, insbesondere eine Düse derart angeordnet, dass der Auslass bzw. die Düse ausgehend von der Ecke in Richtung Manipulatorrauminnerem, insbesondere in Richtung Manipulatorraummitte weist. Ganz besonders bevorzugt ist es, wenn der Auslass bzw. die Düse zu beiden die Ecke bildenden Manipulatorraumwänden den gleichen Winkel einnimmt, insbesondere 45°. Im Hinblick auf die Anordnung der Auslässe, insbesondere Düsen bezogen auf die Vertikalrichtung hat es sich als vorteilhaft herausgestellt, wenn die Auslässe, insbesondere Düsen oberhalb der halben Höhenerstreckung, insbesondere oberhalb einer 2/3-Höhenerstreckung des Manipulatorraums angeordnet sind, gemessen von einem Manipulatorraumboden bis zur den Manipulatorraum gegenüber dem Plenumraum abgrenzenden Membran. Ganz besonders bevorzugt sind die Auslässe bzw. Düsen bezogen auf Manipulatorraumeinbauten, insbesondere Handlingapparaturen zur Handhabung von Pharmazeutika so angeordnet, dass die Düsen sich auf einem Höhenniveau oberhalb der Einbauten befinden, jedoch nicht unmittelbar oberhalb eines Produktweges angeordnet sind, sondern seitlich versetzt hierzu.

Als besonders zweckmäßig hat sich herausgestellt, wenn im Plenumraum pro Quadratmeter innerer Manipulatorraumgrundfläche (Bodenfläche) 0,5 bis 2,0, insbesondere 0,7 bis 1,6 Düsen angeordnet sind und/oder pro Quadratmeter innerer Manipulatorraumgrundfläche (Bodenfläche) des Manipulatorraums zwischen 0,5 und 2,5, insbesondere zwischen 0,8 und 1,5 Düsen.

Als besonders zweckmäßig hat es sich herausgestellt, wenn mindestens eine der vorgenannten Austrittsdüsen, vorzugsweise mehrere der vorgenannten Austrittsdüsen, noch weiter bevorzugt sämtliche sogenannten Austrittsdüsen druckbeaufschlagt sind, derart, dass das diesen über eine Plenumversorgungsleitung bzw. Manipulatorversorgungsleitung zugeführte Medium druckbeaufschlagt ist, insbesondere durch den Einsatz von steriler Druckluft als Trägerluft für H₂0₂-Dampf oder H₂O₂-Nebel, so dass an mindestens einer, vorzugsweise mehreren Austrittsöffnungen der mindestens einen Düse turbulente Strömungsverhältnisse herrschen, die für eine optimierte Verteilung des Wasserstoffperoxiddampfs oder -nebels im Plenumraum bzw. Manipulatorraum Sorge tragen. Ganz besonders bevorzugt ist es, wenn die Austrittsgeschwindigkeit der H₂O₂-haltigen Druckluft an der mindestens einen Austritts-Düse, insbesondere an einer Austrittsöffnung der Düse im Plenumraum, bevorzugt an den mehreren Austrittsdüsen im Plenumraum jeweils zwischen 5,0 m/s und 80 m/s, ganz besonders bevorzugt zwischen 8,0 m/s und 50 m/s beträgt und/oder die Austrittsgeschwindigkeit der H₂O₂-haltigen Druckluft an der mindestens einen Austrittsdüse im Manipulatorraum, bevorzugt an mehreren Austrittsdüsen im Manipulatorraum zwischen 8,0 m/s und 100 m/s, ganz besonders bevorzugt zwischen 12,0 m/s und 80 m/s beträgt.

Wie bereits angedeutet ist eine Ausführungsvariante bevorzugt, bei der die Feinstverteilmittel an eine Trägerluftleitung angeschlossen sind, über die die Feinstverteilmittel mit Trägerluft (Druckluft) von außerhalb des Arbeitsraums versorgt werden. Bevorzugt beträgt dieser Trägerluftvolumenstrom über 40 m³/h, ganz besonders bevorzugt über 70 m³/h, noch weiter bevorzugt zwischen 75 m³/h und 120 m³/h und verteilt sich über die mindestens eine Plenumversorgungsleitung und die mindestens eine Manipulatorversorgungsleitung, versetzt mit Kontaminationsmitteldampf oder - nebel auf dem Plenumraum und dem Manipulatorraum.

Weitere Vorteile, Merkmale und Einzelheiten der Erfindung ergeben sich aus der nachfolgenden Beschreibung eines bevorzugten Ausführungsbeispiels sowie anhand der Zeichnungen.

Diese zeigen in:

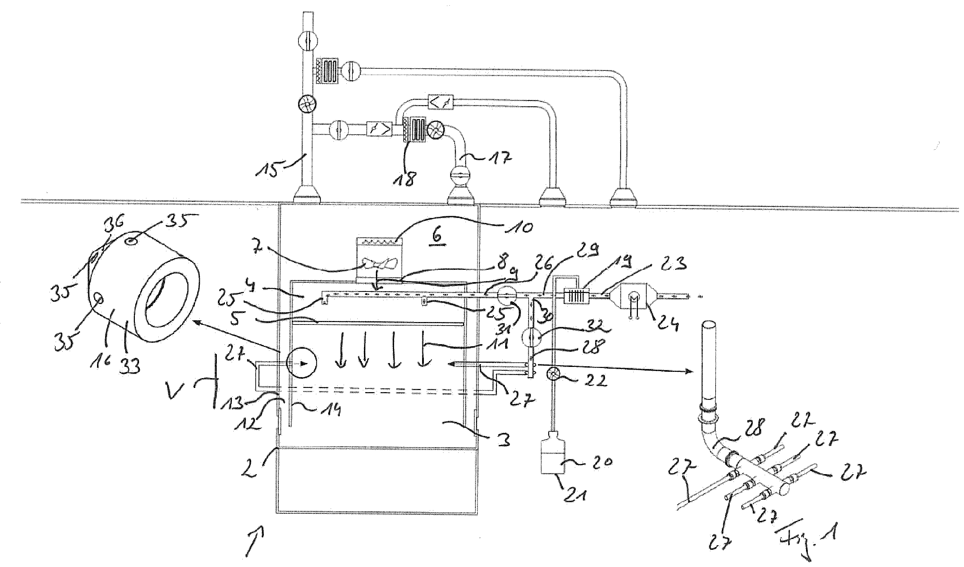

- Fig. 1:: eine nach dem Konzept der Erfindung ausgebildete Dekontaminationsanordnung sowie links eine vergrößerte Darstellung einer bevorzugten Austrittsdüse für ein Dekontaminationsmittel-/Luftgemisch sowie rechts einen Verteilerabschnitt bzw. eine Verteilerflöte zur Aufteilung des Dekontaminationsmittel/Luftvolumenstroms auf mehrere Manipulatorversorgungsleitungen, und

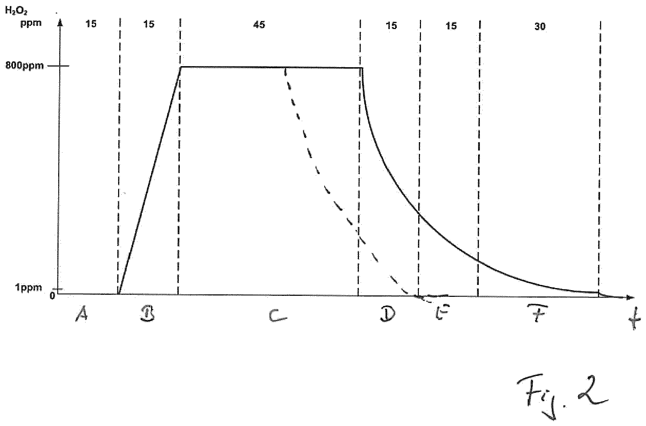

- Fig. 2:: ein Diagramm, welches die Wasserstoffperoxidkonzentration während eines Dekontaminationszyklus zeigt, und zwar im Vergleich zwischen einem bekannten Dekontaminationsverfahren und einem nach dem Konzept der Erfindung ausgebildeten Dekontaminationsverfahren (strichlierte Linie).

In Fig. 1 ist eine nach dem Konzept der Erfindung ausgebildete Dekontaminationsanordnung 1 gezeigt. Diese umfasst einen Isolator 2 für pharmatechnische Anwendungen mit einem Manipulatorraum 3 (Arbeitsraum), in welchem sich nicht gezeigte pharmatechnische Arbeits- und/oder Funktionseinheiten zur Herstellung, Dosierung und/oder Verpackung eines pharmazeutischen Wirkstoffs befinden.

Entlang einer Vertikalen V befindet sich oberhalb des Manipulatorraums 3 ein sogenannter Plenumraum 4 (Luftverteilerraum), der von dem Manipulatorraum 3 über eine horizontal verlaufende Membran 5 getrennt ist. Wiederum entlang der Vertikalen oberhalb des Plenumraums 4 befindet sich ein Umlufterzeugerraum 6 mit einem Umluftgebläse 7 zur Erzeugung eines Umluftvolumenstroms. Der Umlufterzeugerraum 6 ist von dem Plenumraum 4 über einen Hochleistungsschwebstofffilter 8 getrennt, über den der Plenumraum 4 mit einem Luftstrom 9 von dem Umluftgebläse 7 versorgbar ist. Hier beispielhaft in Strömungsrichtung vor dem Umluftgebläse 7 (zusätzlich oder alternativ in Strömungsrichtung hinter dem Umluftgebläse 7) befindet sich ein Katalysator 10, beispielsweise auf MnO₂-Basis zum Abbau von Dekontaminationsmittel, hier H₂O₂, während einer Freispülphase.

Der dem Plenumraum 4 zugeführte Luftstrom strömt, wie erwähnt, über die Membran 5 in den Umlufterzeugerraum 6, wodurch eine in vertikaler Richtung von oben nach unten strömende laminare Luftströmung 11 im Arbeitsraum bzw. Manipulatorraum 3 erzeugt wird, die eine Luftströmungsgeschwindigkeit von 0,45 m/s aufweist. Die laminare Luftströmung 11 strömt, wie erwähnt von oben nach unten und dann in einem unteren Bereich nach außen bzw. seitlich zu mindestens einem Einlass mindestens eines Rückluftkanals 12, der in dem gezeigten Ausführungsbeispiel zwischen zwei durchsichtigen Scheiben 13, 14 ausgebildet ist, über den eine Bedienperson von außen in den Manipulatorraum 3 einsehen kann. Bevorzugt sind diese Scheiben 13, 14 von einem aus Übersichtlichkeitsgründen nicht gezeigten Handschuhport zum manuellen Eingreifen in den Manipulatorraum 3 durchsetzt, was jedoch nicht zwingend ist. Der mindestens eine Rückluftkanal 12 führt entlang der Vertikalen V nach oben in den Umlufterzeugerraum 6, aus welchem dann von dem Umluftgebläse 7 während dessen Betriebs Luft zur Erzeugung des Luftstroms 9 und in der Folge der laminaren Luftströmung 11 angesaugt wird.

Aus dem Umlufterzeugerraum 6 mündet eine Abluftleitung 15 aus, die zumindest während eines Zeitabschnitts einer Dekontaminationsphase zum Abführen eines Trägerluftvolumenstroms in Richtung Umgebung geöffnet ist. Ferner mündet in den Umlufterzeugerraum 6 eine Frischluftleitung 17 zum Zuführen von steriler konditionierter Luft, deren Temperatur und Feuchtigkeitsgehalt von entsprechenden Konditioniermitteln 18 auf die gewünschten Werte geregelt wird.

Um den Isolator 2 während eines Dekontaminationsbetriebszustandes zu dekontaminieren umfasst die Dekontaminationsanordnung Feinstverteilmittel 19, hier beispielhaft in Form eines einzigen Blitzverdampfer zum Verdampfen von Dekontaminationsmittel 20, hier H₂O₂ aus einem Vorrat 21. Das Dekontaminationsmittel 20 wird über eine Pumpe 22 dem Verdampfer 19 zugeführt und dort verdampft, wobei der Dekontaminationsmitteldampf von über eine externe Trägerluftleitung 23 zugeführter Trägerluft mitgerissen wird. Die Trägerluft wird von extern zugeführt und wird vor Eintritt in den Isolator 2, insbesondere durch das Vorsehen mindestens eines entsprechenden Hochleistungsschwebstofffilters sterilisiert. Die Trägerluft ist bevorzugt temperiert, insbesondere auf eine Temperatur von 45°C und getrocknet. Hierzu ist eine entsprechende Konditioniereinrichtung 24 in der Trägerluftleitung 23 integriert.

In dem konkreten Ausführungsbeispiel befinden sich die Feinstverteilmittel 19 außerhalb der Funktionsräume des Isolators 2, was bevorzugt ist - alternativ ist es möglich diese in einem solchen Funktionsraum anzuordnen.

Mit den Feinstverteilmitteln 19 gasleitend verbunden sind mehrere Plenumversorgungsleitungen 25, die unmittelbar in den Plenumraum 4 ausmünden, so dass das Dekontaminationsmittel-/Luftgemisch unmittelbar in den Plenumraum 4 in einem Bereich oberhalb der Membran 5 ausgeblasen werden kann. Die Plenumversorgungsleitungen 25 münden aus einer Plenumversorgungsverteilerleitung 26 aus. Bevorzugt befinden sich endseitig an den Plenumversorgungsleitungen 25 Düsen.

Ferner gasleitend mit den Feinstverteilmitteln 19, hier beispielhaft dem einzigen bzw. gemeinsamen Blitzverdampfer sind mehrere Manipulatorversorgungsleitungen 27 verbunden, die seitlich, d.h. im Bereich der inneren Manipulatorraumwände unmittelbar in den Manipulatorraum 3 ausmünden, so dass auch in den Manipulatorraum 3 unmittelbar das Dekontaminationsmittel-/Luftgemisch einblasbar ist. Die Manipulatorversorgungsleitungen 27 münden aus einer Manipulatorversorgungsverteilerleitung 28 (Verteilerflöte) aus. Die Manipulatorversorgungsverteilerleitung 28 und die Plenumversorgungsverteilerleitung 26 werden versorgt von einer gemeinsamen Versorgungsleitung 29, die somit sowohl einen Bestandteil der Plenumversorgungsleitungen 25 als auch der Manipulatorversorgungsleitungen 27 bildet. Die unmittelbar an die Feinstverteilermittel 19 angeschlossene Versorgungsleitung 29 teilt sich an einem Abzweig 30 auf in die Plenumversorgungsleitungen 25 und die Manipulatorversorgungsleitungen 27 bzw. eine Hauptplenumversorgungsleitung (Plenumversorgungsverteilerleitung) und eine Hauptmanipulatorleitung (Manipulatorversorgungsverteilerleitung). In beiden Hauptverteilungsleitungen sind Ventilmittel vorgesehen. Konkret ist den Plenumversorgungsleitungen 25 ein Plenumventil 31 zugeordnet und den Manipulatorversorgungsleitungen 27 ein Manipulatorventil 32. Grundsätzlich reicht eines von beiden Ventilen 31, 32 aus, um eine Störluftströmung ausgehend vom Plenumraum 4 über die Plenumversorgungsleitungen 25 und die Manipulatorversorgungsleitungen 28 in den Manipulatorraum 3 zu verhindern. Auch eine solche Lösung ist realisierbar. Bevorzugt ist jedoch die gezeigte Variante mit zwei Ventilen. Durch das Vorsehen der Ventile, von denen bevorzugt zumindest eines als Proportionalventil ausgebildet ist, können Plenumraum 4 und Manipulatorraum 3 getaktet mit Dekontaminationsmittel-/Luftgemisch versorgt werden, d.h. alternierend jeweils mehrfach hintereinander. Zusätzlich oder alternativ ist es möglich das Volumenstromverhältnis der über die Plenumversorgungsleitungen 25 und die Manipulatorversorgungsleitungen 28 auströmenden Volumenströme zu variieren, wobei es besonders bevorzugt ist das Volumenstromverhältnis während der Dekontaminationsphase zu verändern.

Anstelle der gezeigten Ausführungsvariante ist es denkbar, die Manipulatorversorgungsleitungen 28 und die Plenumversorgungsleitungen 25 über separate Verdampfer oder Zerstäuber mit einem Dekontaminationsmittel-/Luftgemisch zu versorgen. Bevorzugt ist die Variante mit einem gemeinsamen Verdampfer oder Zerstäuber, wobei in diesem Fall bevorzugt mindestens ein Ventil zur Unterbindung der erwähnten Störluftströmung vorgesehen ist.

In der Zeichnungsebene gemäß Fig. 1 links ist eine bevorzugte Ausführungsform einer endseitigen Düse 33 gezeigt, die Einströmung eines Dekontaminationsmittel-/Luftgemisches erfolgt in der Zeichnungsebene von links nach rechts, d.h. in die gezeigte Eingangsöffnung 34 hinein und dann über diverse Austrittsöffnungen 35 in den jeweiligen Raum, wobei sowohl an einem vorderen Konusabschnitt 36 als auch an einem dahinterliegenden Zylinderabschnitt 16 jeweils Austrittsöffnungen 35 vorgesehen sind, die in unterschiedliche Richtungen weisen. Düsen 33, bevorzugt wie gezeigt können sowohl endseitig an den Manipulatorversorgungsleitungen 27 als auch an den Plenumversorgungsleitungen 25 vorgesehen werden.

In der Zeichnungsebene rechts ist vergrößert eine Ausführungsvariante der Manipulatorversorgungsverteilerleitung 28 gezeigt, aus welcher endseitig diverse Manipulatorversorgungsleitungen 27 ausmünden, die dann unmittelbar im Manipulatorraum 3 enden.

Während einer Dekontaminationsphase ist die Abluftleitung 15 geöffnet und Trägerluft strömt über die Trägerleitung 23 zu den Feinstverteilmitteln 19 und wird dort mit Dekontaminationsmittel in Dampf- oder Nebelform beaufschlagt, wobei das resultierende Dekontaminationsmittel-/Luftgemisch sowohl unmittelbar in den Plenumraum 4 als auch in den Manipulatorraum 3 einströmt. Aufgrund der geöffneten Abluftleitung 15 resultiert eine Konvektion und das Gemisch verteilt sich im Plenumraum 4, im Arbeitsraum, in den Rückluftkanälen 12 sowie im Umlufterzeugerraum 6. Das Umluftgebläse 7 ist während des größten Zeitabschnitts dieser Dekontaminationsphase ausgeschaltet, um einen Abbau von Dekontaminationsmittel im Katalysator 10 zu verhindern. Bei einer Ausführungsvariante ohne den, wie gezeigt angeordneten Katalysator 10 könnte das Umluftgebläse 7 betrieben werden, was jedoch bevorzugt auch deshalb verhindert wird, um ein zu starkes Aufladen des Hochleistungsschwebstofffilters 8 zu verhindern. Auf die Dekontaminationsphase, die neben der Zuführphase von Dekontaminationsmittel-/Luftgemisch eine Einwirkzeit beinhaltet wird das Umluftgebläse 7 zum Freispülen des Isolators 2 im Umluftbetrieb betrieben. Bei Bedarf kann gleichzeitig konditionierte Frischluft über die Zuluftleitung 17 zugeführt und dann entsprechend Abluft über die Abluftleitung 15 abgeführt werden. Auf die Freispülphase folgt eine Arbeitsphase, in der die nicht gezeigten Arbeits- und/oder Funktionseinheiten im Manipulatorraum 3 betrieben werden. Während dieses Betriebs wird das Umluftgebläse 7 betrieben und erzeugt, wie erläutert den laminaren Luftstrom in vertikaler Richtung. Störluftströmungen über die Manipulatorversorgungsleitungen 27 werden aufgrund entsprechender Ventilmittel verhindert.

Zum Nachweis der überraschend großen Vorteile der erfindungsgemäßen Vorrichtung und des erfindungsgemäßen Verfahrens im Hinblick auf die Reduzierung der (Dekontaminations-)Zykluszeit und des H₂O₂-Verbrauchs hat die Anmelderin diverse Versuche durchgeführt.

Hierzu hat die Anmelderin eine in Fig. 1 gezeigte Dekontaminationsanordnung genutzt, wobei zur Simulation einer Stand der Technik-Dekontaminationsanordnung die Zuleitung von Dekontaminationsmittel unmittelbar in den Manipulatorraum 3 unterbrochen bzw. versperrt wurde und ausschließlich Dekontaminationsmitteldampf (wie im Stand der Technik) in den Plenumraum 4 geleitet wurde. Zur Feststellung der Ergebnisse gemäß einem erfindungsgemäßen Verfahren wurde gleichzeitig H₂O₂-Dampf in den Manipulatorraum 3 sowie den Plenumraum 4 ausgeleitet. Dabei hat die Anmelderin festgestellt, dass der Wasserstoffperoxidverbrauch bei einer erfindungsgemäßen Betriebsweise, bei der Wasserstoffperoxiddampf sowohl in den Plenumraum als auch in den Manipulatorraum eingeleitet wurde verglichen mit der Stand der Technik-Variante von 773 g auf 473 g, d.h. um 43 % reduziert werden konnte. Zudem konnte, wie aus der im Folgenden noch zu erläuternden Fig. 2 ersichtlich ist, die H₂O₂-Injektionsphase C von 57 min auf 33 min, d.h. um 42 % reduziert werden. Darüber hinaus konnte die Freispül-, d.h. Belüftungszeit deutlich verkürzt werden, was aus einem Vergleich der strichlierten Linie mit der durchgezogenen, abfallenden Linie ersichtlich wird. Letzteren Effekt erklärt sich die Anmelderin durch die reduzierte absolulte Menge an benötigtem H₂O₂.

In dem zuvor angesprochenen Diagramm in Fig. 2 ist auf der Y-Achse die H₂O₂-Konzentration im Manipulatorraum in ppm angegeben, diese ist über die Zeit (t) (X-Achse) aufgetragen. Die Zeitachse ist in mehrere Funktionszeiten bzw. -abschnitte unterteilt. Bei der Phase A handelt es sich um eine Vorkonditionierungsphase, bei der das Isolatorinnenvolumen mit konditionierter, d.h. temperierter und entfeuchteter Luft vorgespült wird. Daraufhin folgt eine Konditionierungsphase B von 15 min, in welcher die Wasserstoffperoxidkonzentration auf die gewünschte Konzentration von 800 ppm erhöht wurde. Daraufhin erfolgt die Dekontaminationsphase C, die bei einem erfindungsgemäßen Verfahren nur bis zu Beginn der strichlierten Linie dauert und verglichen mit einem Stand der Technik-Verfahren (durchgezogene Linie) deutlich verkürzt ist. Die Phasen D, E und F sind Freispülphasen im Stand der Technik - diese beginnen bei einem erfindungsgemäßen Verfahren zu einem früheren Zeitpunkt und sind aus Übersichtlichkeitsgründen für das erfindungsgemäße Verfahren (strichlierte Linie) an der X-Ache nicht angezeichnet - im Prinzip ergibt sich eine Verschiebung dieser Phasen nach links, verglichen mit dem Stand der Technik-Verfahren.

Die vorerwähnten Vorteile werden im Wesentlichen auf die erfindungsgemäß verbesserte Gasverteilung zurückgeführt und die bevorzugte Realisierung von turbulenten Strömungsverhältnissen sowohl im Plenumraum als auch im Manipulatorraum.

Zur Stütze vorgenannter Versuchsergebnisse hat die Anmelderin auch die Dezimalreduktionszeit (D-Wert) an unterschiedlichen Manipulatorraumpositionen verglichen zwischen der Stand der Technik-Ausführung und der erfindungsgemäßen Ausführung. Bei der Dezimalreduktionszeit (D-Wert) handelt es sich um die Zeit, in der 9/10 der Population eines biologischen Indikators (hier wurde selbstverständlich bei den Vergleichsversuchen der gleiche Bioindikator an den gleichen Positionen eingesetzt) abstirbt. An den sonstigen Parametern, wie die Temperatur usw. wurde dabei nichts verändert. Die Ergebnisse bestätigen die Vorteilhaftigkeit einer erfindungsgemäßen Vorrichtung (Dekontaminationsanordnung) und eines erfindungsgemäßen Verfahrens gegenüber der Stand der Technik-Ausführung. Lediglich beispielhaft seien hier zwei Versuchsergebnisse herausgegriffen. Untersucht wurde der D-Wert bezogen auf zwei unterschiedliche, kritische Bioindikatorpositionen. Bei der erfindungsgemäßen Ausführung konnte der D-Wert von 5,2 min bzw. 5,7 min bei der Stand der Technik-Variante jeweils auf unter 2 min bei der erfindungsgemäßen Variante reduziert werden.

### Bezugszeichenliste

- 1: Dekontaminationsanordnung
- 2: Isolator
- 3: Manipulatorraum
- 4: Plenumraum
- 5: Membran
- 6: Umlufterzeugerraum
- 7: Umluftgebläse
- 8: Hochleistungsschwebstofffilter
- 9: Luftstrom
- 10: Katalysator
- 11: laminare Luftströmung
- 12: Rückluftkanal
- 13: Scheibe
- 14: Scheibe
- 15: Abluftkanal
- 16: Zylinderabschnitt
- 17: Frischluftkanal
- 18: Konditioniermittel
- 19: Feinstverteilmittel
- 20: Dekontaminationsmittel
- 21: Vorrat
- 22: Pumpe
- 23: Trägerluftleitung
- 24: Konditioniereinrichtung
- 25: Plenumversorgungsleitungen
- 26: Plenumversorgungsverteilerleitung
- 27: Manipulatorversorgungsleitungen
- 28: Manipulatorversorgungsverteilerleitung
- 29: gemeinsame Versorgungsleitung
- 30: Abzweigung
- 31: Plenumventil
- 32: Manipulatorventil
- 33: Düse
- 34: Eingangsöffnung
- 35: Austrittsöffnungen
- 36: Konusabschnitt

- V: Vertikalrichtung (Vertikale)

## Patentansprüche

1. Dekontaminationsanordnung (1), umfassend einen Isolator (2) mit einem Manipulatorraum (3) und einem oberhalb des Manipulatorraums (3) angeordneten Plenumraum (4), der über mindestens ein in einem Umlufterzeugerraum (6) angeordnetes Umluftgebläse (7) mit einem Luftstrom (9) versorgbar ist, wobei zwischen Plenumraum (4) und Manipulatorraum (3) eine Membran (5) zum Erzeugen einer laminaren Luftströmung (11) im Manipulatorraum (3) während eines Arbeitsbetriebszustandes angeordnet ist, wobei der Manipulatorraum (3) luftleitend mit dem Umlufterzeugerraum (6) über einen Rückluftkanal (12) verbunden ist, weiter umfassend Feinstverteilmittel (19) zum Erzeugen eines Dekonatminationsmittel-/Luftgemisches auf Basis eines flüssigen Dekontaminationsmittelvorrats, wobei die Feinstverteilmittel (19) über mindestens eine in den Plenumraum (4) einmündende Plenumversorgungsleitung (25) mit dem Plenumraum (4) verbunden sind, um den Plenumraum (4) unmittelbar mit dem Dekontaminationsmittel-/Luftgemisch in einem Dekontaminationsbetriebszustand zu versorgen,
**dadurch gekennzeichnet,**
**dass** die Feinstverteilmittel (19) über mindestens eine Manipulatorversorgungsleitung (27) unmittelbar mit dem Manipulatorraum (3) verbunden sind, um den Manipulatorraum (3) in dem Dekontaminationsbetriebszustand unmittelbar mit dem Dekontaminationsmittel-/Luftgemisch zu versorgen.

2. Dekontaminationsanordnung nach Anspruch 1,
**dadurch gekennzeichnet,**
**dass** mehrere an unterschiedlichen Stellen in den Plenumraum (4) ausmündende Plenumversorgungsleitungen (25) und/oder mehrere an unterschiedlichen Stellen in den Manipulatorraum (3) ausmündende Manipulatorversorgungsleitungen (27) vorgesehen sind.

3. Dekontaminationsanordnung nach einem der 1 oder 2,
**dadurch gekennzeichnet,**
**dass** der Plenumraum (4) und der Manipulatorraum (3) gleichzeitig über die mindestens eine Plenumversorgungsleitung (25) und die mindestens eine Manipulatorversorgungsleitung (27) mit dem Dekontaminationsmittel-/Luftgemisch versorgbar sind.

4. Dekontaminationsanordnung nach einem der vorhergehenden Ansprüche,
**dadurch gekennzeichnet,**
**dass** der mindestens einen Plenumversorgungsleitung (25) und/oder der mindestens einen Manipulatorversorgungsleitung (27) Ventilmittel zugeordnet sind, mit denen ein Strömen von Luft während des Arbeitsbetriebszustandes aus dem Plenumraum (4) über die mindestens eine Plenumversorgungsleitung (25) und die Manipulatorversorgungsleitung (27) verhinderbar ist.

5. Dekontaminationsanordnung nach einem der vorhergehenden Ansprüche,
**dadurch gekennzeichnet,**
**dass** der mindestens einen Plenumversorgungsleitung (25) ein in einem Bereich zwischen den Feinstverteilmitteln (19) und einem Plenumversorgungsleitungsauslass angeordnetes Plenumventil (31) zugeordnet ist und/oder dass der mindestens einen Manipulatorversorgungsleitung (27) ein in einem Bereich zwischen den Feinstverteilmitteln (19) und einem Bereich einem Plenumversorgungsleitungsauslass angeordnetes Manipulatorventil (32) zugeordnet ist.

6. Dekontaminationsanordnung nach Anspruch 5,
**dadurch gekennzeichnet,**
**dass** das Plenumventil (31) und/oder das Manipulatorventil (32) derart von Steuermitteln ansteuerbar sind, dass Plenumraum (4) und Manipulatorraum (3) gleichzeitig mit dem Dekontaminationsmittel-/Luftgemisch versorgbar sind und/oder derart, dass Plenumraum (4) und Manipulatorraum (3) nacheinander, insbesondere mehrfach alternierend, mit dem Dekontaminationsmittel-/Luftgemisch versorgbar sind und/oder derart, dass ein Volumenstromverhältnis der in den Plenumraum (4) und den Manipulatorraum (3) einströmenden Dekontaminationsmittel-/Luftgemischvolumenströme einstellbar, bevorzugt während des Dekontaminationsbetriebszustandes veränderbar ist.

7. Dekontaminationsanordnung nach einem der vorhergehenden Ansprüche,
**dadurch gekennzeichnet,**
**dass** die Feinstverteilmittel (19) einen gemeinsamen Verdampfer oder einen gemeinsamen Zerstäuber umfassen, der an die Plenumversorgungsleitung (25) und die Manipulatorversorgungsleitung (27) angeschlossen ist, oder dass die Feinstverteilmittel (19) separate Verdampfer oder Zerstäuber zum Beaufschlagen der Plenumversorgungsleitung (25) und der Manipulatorversorgungsleitung (27) aufweisen.

8. Dekontaminationsanordnung nach einem der vorhergehenden Ansprüche,
**dadurch gekennzeichnet,**
**dass** zwischen dem Plenumraum (4) und dem, bevorzugt oberhalb des Plenumraums (4) angeordneten, Umlufterzeugerraum (6) ein Hochleistungsschwebstofffilter (8), insbesondere ein ULPA-Filter oder ein HEPA-Filter angeordnet ist, über den der Plenumraum (4) mit dem von dem Umluftgebläse (7) erzeugten Luftstrom (9) beaufschlagbar ist.

9. Dekontaminationsanordnung nach einem der vorhergehenden Ansprüche,
**dadurch gekennzeichnet,**
**dass** dem über Steuermittel ansteuerbaren Umluftgebläse (7) ein Katalysator (10) zum Abbau von Dekontaminationsmittel (20) im Umluftbetrieb zugeordnet ist.

10. Dekontaminationsanordnung nach Anspruch 9,
**dadurch gekennzeichnet,**
**dass** der Katalysator (10) stationär relativ zu dem Umluftgebläse (7) angeordnet ist und die Steuermittel das Umluftgebläse (7) derart ansteuernd ausgebildet sind, dass das Umluftgebläse (7) während des Dekontaminationsbetriebszustandes ausgeschaltet ist, oder dass zwischen dem Katalysator (10) und dem Umluftgebläse, (7) bevorzugt durch Verstellen des Katalysators (10) oder von diesem zugeordneten Luftleitmitteln ein Bypass erzeugbar ist und dass die Steuermittel das Umluftgebläse (7) derart ansteuernd ausgebildet sind, dass dieses bei erzeugtem Bypass während des Dekontaminationsbetriebszustandes betrieben wird.

11. Dekontaminationsanordnung nach einem der vorhergehenden Ansprüche,
**dadurch gekennzeichnet,**
**dass** die mindestens eine Plenumversorgungsleitung (25) und/oder die mindestens eine Manipulatorversorgungsleitung (27) über jeweils eine Düse (33) in den Plenumraum (4) bzw. den Manipulatorraum (3) ausmündet, wobei die Düse (33) mehre in unterschiedliche Richtungen weisende Austrittsöffnungen (35) aufweist, die bevorzugt auf einen Konusabschnitt (36) und einen zum Konusabschnitt (36) benachbarten Zylinderabschnitt verteilt sind.

12. Dekontaminationsanordnung nach einem der vorhergehenden Ansprüche,
**dadurch gekennzeichnet,**
**dass** die Feinstverteilmittel (19) an eine Trägerluftleitung (23) zum Versorgen der Feinstverteilmittel (19) während der Dekontaminationsphase mit Trägerluft von außerhalb des Arbeitsraums zur Aufnahme von Dekontaminationsmitteldampf oder zum Zerstäuben von Dekontaminationsmittel angeschlossen sind.

13. Dekontaminationsanordnung nach einem der vorhergehenden Ansprüche,
**dadurch gekennzeichnet,**
**dass** die mindestens Manipulatorversorgungsleitung (27) im Bereich einer Manipulatorraumseitenwand in den Manipulatorraum (3) einmündet und/oder direkt vertikal unterhalb des Manipulatorversorgungsleitungsendes keine pharmatechnische Arbeits- oder Funktionseinheit angeordnet ist.

14. Verfahren zum Betreiben einer Dekontaminationsanordnung (1), bevorzugt nach einem der vorhergehenden Ansprüche, umfassend einen Isolator (2) mit einem Manipulatorraum (3) und einem oberhalb des Manipulatorraums (3) angeordneten Plenumraum (4), der über mindestens ein in einem Umlufterzeugerraum (6) angeordneten Umluftgebläse (7) mit einem Luftstrom (9) versorgt wird, wobei zwischen Plenumraum (4) und Manipulatorraum (3) eine Membran (5) zum Erzeugen einer, bevorzugt vertikalen, laminaren Luftströmung (11) im Manipulatorraum (3) während eines Arbeitsbetriebszustandes angeordnet ist, wobei der Manipulatorraum (3) luftleitend mit dem Umlufterzeugerraum (6) über einen Rückluftkanal (12) verbunden ist, der zwischen zwei durchsichtigen Scheiben (13, 14) angeordnet ist, weiter umfassend Feinstverteilmittel (19) zum Erzeugen eines Dekontaminationsmittel-/Luftgemisches auf Basis eines flüssigen Dekontaminationsmittelvorrats, wobei die Feinstverteilmittel (19) über mindestens eine in den Plenumraum (4) einmündende Plenumversorgungsleitung (25) mit dem Plenumraum (4) verbunden ist, über die der Plenumraum (4) unmittelbar mit dem Dekontaminationsmittel-/Luftgemisch beaufschlagt wird,
**dadurch gekennzeichnet,**
**dass** die Feinstverteilmittel (19) über mindestens eine Manipulatorversorgungsleitung (27) unmittelbar dem Manipulatorraum (3) verbunden sind, über die der Manipulatorraum (3) in dem Dekontaminationsbetriebszustand unmittelbar mit Dekontaminationsmittel-/Luftgemisch versorgt wird.

15. Verfahren nach Anspruch 14,
**dadurch gekennzeichnet,**
**dass** Plenumraum (4) und Manipulatorraum (3) gleichzeitig mit dem Dekontaminationsmittel-/Luftgemisch beaufschlagt werden und/oder dass Plenumraum (4) und Manipulatorraum (3) nacheinander, insbesondere mehrfach alternierend, mit dem Dekontaminationsmittel-/Luftgemisch beaufschlagt werden und/oder dass das ein Volumenstromverhältnis der in den Plenumraum (4) und den Manipulatorraum (3) einströmenden Dekontaminationsmittel-/Luftgemischvolumenströme eingestellt, bevorzugt während des Dekontaminationsbetriebszustandes verändert wird.

16. Verfahren nach einem der Ansprüche 14 oder 15,
**dadurch gekennzeichnet,**
**dass** ein Durchgang von der mindestens einen Plenumversorgungsleitung (25) zu der mindestens einen Manipulatorversorgungsleitung (27) während des Arbeitsbetriebszustandes geschlossen ist, um eine Störung der laminaren Luftströmung (11) im Manipulatorraum (3) durch ohne die Schließung von dem Plenumraum (4) über die Plenumversorgungsleitung (25) und die Manipulatorversorgungsleitung (27) in den Manipulatorraum (4) einströmende Luft zu verhindern.

## Claims

1. A decontamination assembly (1), comprising an isolator (2) having a manipulator chamber (3) and a plenum chamber (4) which is disposed above the manipulator chamber (3) and which can be supplied with an air flow (9) via at least one circulation fan (7) disposed in a circulating-air generation chamber, a membrane (5) for generating a laminar air flow (11) in the manipulator chamber (3) during a working operating state being disposed between the plenum chamber (4) and the manipulator chamber (3), the manipulator chamber (3) being connected to the circulating-air generation chamber (6) in an air-conducting manner via a return-air channel (12), further comprising ultrafine distributing means (19) for producing a decontaminant/air mixture based on a liquid decontaminant stock, the ultrafine distributing means (19) being connected to the plenum chamber (4) via at least one plenum supply line (25) leading into the plenum chamber (4) so as to directly supply the plenum chamber (4) with the decontaminant/air mixture when in a decontamination operating state,
**characterized in that**
the ultrafine distributing means (19) are connected directly to the manipulator chamber (3) via at least one manipulator supply line (27) so as to directly supply the manipulator chamber (3) with the decontaminant/air mixture when in the decontamination operating state.

2. The decontamination assembly according to claim 1,
**characterized in that**
multiple plenum supply lines (25) leading into the plenum chamber (4) at different locations and/or multiple manipulator supply lines (27) leading into the manipulator chamber (3) at different locations are provided.

3. The decontamination assembly according to any one of claims 1 or 2,
**characterized in that**
the plenum chamber (4) and the manipulator chamber (3) can be simultaneously supplied with the decontaminant/air mixture via the at least one plenum supply line (25) and the at least one manipulator supply line (27).

4. The decontamination assembly according to any one of the preceding claims,
**characterized in that**
valve means by means of which air can be prevented from flowing out of the plenum chamber (4) via the at least one plenum supply line (25) and the manipulator supply line (27) during the working operating state are assigned to the at least one plenum supply line (25) and/or to the at least one manipulator supply line (27).

5. The decontamination assembly according to any one of the preceding claims,
**characterized in that**
a plenum valve (31) disposed in an area between the ultrafine distributing means (19) and a plenum supply line outlet is assigned to the at least one plenum supply line (25) and/or that a manipulator valve (32) disposed in an area between the ultrafine distributing means (19) and a plenum supply line outlet is assigned to the at least one manipulator supply line (27).

6. The decontamination assembly according to claim 5,
**characterized in that**
the plenum valve (31) and/or the manipulator valve (32) can be controlled by control means in such a manner that the plenum chamber (4) and the manipulator chamber (3) can be supplied with the decontaminant/air mixture simultaneously and/or in such a manner that the plenum chamber (4) and the manipulator chamber (3) can be supplied with the contaminant/air mixture one after the other, in particular in a repeated alternating manner, and/ or in such a manner that a volumetric flow rate of the decontaminant/air-mixture volumetric flows flowing into the plenum chamber (4) and into the manipulator chamber (3) can be adjusted, preferably changed during the decontamination operating state.

7. The decontamination assembly according to any one of the preceding claims,
**characterized in that**
the ultrafine distributing means (19) comprise a shared evaporator or a shared nebulizer which is connected to the plenum supply line (25) and to the manipulator supply line (27) or that the ultrafine distributing means (19) have separate evaporators or nebulizers for supplying the plenum supply line (25) and the manipulator supply line (27).

8. The decontamination assembly according to any one of the preceding claims,
**characterized in that**
a high-performance filter (8) for suspended particles, in particular an ULPA filter or a HEPA filter, via which the air flow (9) generated by the circulation fan (7) can be applied to the plenum chamber (4) is disposed between the plenum chamber (4) and the circulating-air generation chamber (6), which is preferably disposed above the plenum chamber (4).

9. The decontamination assembly according to any one of the preceding claims,
**characterized in that**
a catalytic converter (10) for degrading decontaminant (20) during circulating-air operation is assigned to the circulation fan (7) controllable via controlling means.

10. The decontamination assembly according to claim 9,
**characterized in that**
the catalytic converter (10) is disposed stationary relative to the circulation fan (7) and the control means are configured to control the circulation fan (7) in such a manner that the circulation fan (7) is switched off during the decontamination operating state, or that a bypass can be established between the catalytic converter (10) and the circulation fan (7), preferably by shifting the catalytic converter (10) or by shifting air conducting means assigned thereto, and that the control means are configured to control the circulation fan (7) in such a manner that it is operated during the decontamination operating state when the bypass is established.

11. The decontamination assembly according to any one of the preceding claims,
**characterized in that**
the at least one plenum supply line (25) and/or the at least one manipulator supply line (27) lead into the plenum chamber (4) and into the manipulator chamber (3), respectively, via a nozzle (33), each nozzle (33) having multiple outlet openings (35), which point in different directions and are preferably distributed across a cone section (36) and cylinder section adjacent to the cone section (36).

12. The decontamination assembly according to any one of the preceding claims,
**characterized in that**
the ultrafine distributing means (19) are connected to a carrier air line (23) for supplying the ultrafine distributing means (19) with carrier air from outside the working chamber during the decontamination phase, the carrier air serving to absorb decontaminant vapor or to nebulize decontaminant.

13. The decontamination assembly according to any one of the preceding claims,
**characterized in that**
the at least one manipulator supply line (27) leads into the manipulator chamber (3) in the area of a manipulator chamber side wall and/or that no pharmaceutical working unit or functional unit is disposed directly vertically below the manipulator supply line end.

14. A method for operating a decontamination assembly (1), preferably according to any one of the preceding claims, comprising an isolator (2) having a manipulator chamber (3) and a plenum chamber (4) which is disposed above the manipulator chamber (3) and which is supplied with an air flow (9) via at least one circulation fan (7) disposed in a circulating-air generation chamber (6), a membrane (5) for generating a preferably vertical laminar air flow (11) in the manipulator chamber (3) during a working operating state being disposed between the plenum chamber (4) and the manipulator chamber (3), the manipulator chamber (3) being connected to the circulating-air generation chamber (6) in an air-conducting manner via a return-air channel (12) which is disposed between two transparent plates (13, 14), further comprising ultrafine distributing means (19) for producing a decontaminant/air mixture based on a liquid decontaminant stock, the ultrafine distributing means (19) being connected to the plenum chamber (4) via at least one plenum supply line (25) which leads into the plenum chamber (4) and via which the plenum chamber (4) is directly supplied with the decontaminant/air mixture,
**characterized in that**
the ultrafine distributing means (19) are connected directly to the manipulator chamber (3) via at least one manipulator supply line (27) via which the manipulator chamber (3) is directly supplied with the decontaminant/air mixture when in the decontamination operating state.

15. The method according to claim 14,
**characterized in that**
the plenum chamber (4) and the manipulator chamber (3) are supplied with the decontaminant/air mixture simultaneously and/or **in that** the plenum chamber (4) and the manipulator chamber (3) are supplied with the contaminant/air mixture one after the other, in particular in a repeated alternating manner, and/ or **in that** a volumetric flow rate of the decontaminant/air-mixture volumetric flows flowing into the plenum chamber (4) and into the manipulator chamber (3) are adjusted, preferably changed during the decontamination operating state.

16. The method according to any one of claims 14 or 15,
**characterized in that**
a passage from the at least one plenum supply line (25) to the at least one manipulator supply line (27) is closed during the working operating state so as to prevent air flowing from the plenum chamber (4) into the manipulator chamber (3) via the plenum supply line (25) and the manipulator supply line (27) when the passage is not closed from disturbing the laminar air flow (11) in the manipulator chamber (3).

## Revendications

1. Système de décontamination (1), comprenant un isolateur (2) ayant une chambre de manipulation (3) et une chambre de plénum (4) qui est disposée au-dessus de la chambre de manipulation (3) et qui peut être alimentée en un flux d'air (9) à l'aide d'au moins un ventilateur de circulation (7) disposé dans une chambre de production de circulation d'air (6), une membrane (5) destinée à produire un flux d'air (11) laminaire dans la chambre de manipulation (3) pendant un état de fonctionnement de travail étant disposée entre la chambre de plénum (4) et la chambre de manipulation (3), la chambre de manipulation (3) étant reliée à la chambre de production de circulation d'air (6) de manière à conduire de l'air par un canal d'air de retour (12), comprenant en outre des moyens de distribution ultrafins (19) destinés à produire un mélange décontaminant-air sur la base d'une réserve de décontaminant liquide, les moyens de distribution ultrafins (19) étant reliés à la chambre de plénum (4) par au moins une conduite d'alimentation de plénum (25) débouchant dans la chambre de plénum (4) afin d'alimenter directement la chambre de plénum (4) en mélange décontaminant-air à un état de fonctionnement de décontamination,
**caractérisé en ce que**
les moyens de distribution ultrafins (19) sont reliés directement à la chambre de manipulation (3) par au moins une conduite d'alimentation de manipulateur (27) afin d'alimenter directement la chambre de manipulation (3) en mélange décontaminant-air à l'état de fonctionnement de décontamination.

2. Système de décontamination selon la revendication 1,
**caractérisé en ce que**
plusieurs conduites d'alimentation de plénum (25) débouchant dans la chambre de plénum (4) à différent endroits et/ou plusieurs conduites d'alimentation de manipulateur (27) débouchant dans la chambre de manipulation (3) à différents endroits sont prévues.

3. Système de décontamination selon l'une quelconque des revendications 1 ou 2,
**caractérisé en ce que**
la chambre de plénum (4) et la chambre de manipulation (3) peuvent être alimentées simultanément en mélange décontaminant-air par l'au moins une conduite d'alimentation de plénum (25) et l'au moins une conduite d'alimentation de manipulateur (27).

4. Système de décontamination selon l'une quelconque des revendications précédentes,
**caractérisé en ce que**
des moyens de soupape par lesquels l'air peut être empêché de couler à partir de la chambre de plénum (4) par l'au moins une conduite d'alimentation de plénum (25) et par la conduite d'alimentation de manipulateur (27) pendant l'état de fonctionnement de travail sont assignés à l'au moins une conduite d'alimentation de plénum (25) et/ou à l'au moins une conduite d'alimentation de manipulateur (27).

5. Système de décontamination selon l'une quelconque des revendications précédentes,
**caractérisé en ce qu'**
une soupape de plénum (31) disposée dans une région entre les moyens de distribution ultrafins (19) et une sortie de la conduite d'alimentation de plénum est assignée à l'au moins une conduite d'alimentation de plénum (25) et/ou qu'une soupape de manipulation (32) disposée dans une région entre les moyens de distribution ultrafins (19) et une sortie de la conduite d'alimentation de plénum est assignée à l'au moins une conduite d'alimentation de manipulateur (27).

6. Système de décontamination selon la revendication 5,
**caractérisé en ce que**
la soupape de plénum (31) et/ou la soupape de manipulation (32) peuvent être commandées par des moyens de commande de telle manière que la chambre de plénum (4) et la chambre de manipulation (3) peuvent être alimentées simultanément en mélange décontaminant-air et/ou de telle manière que la chambre de plénum (4) et la chambre de manipulation (3) peuvent être alimentées en mélange contaminant/air l'une après l'autre, notamment plusieurs fois en alternance, et/ou de telle manière qu'un débit volumique des flux volumiques en mélange décontaminant-air coulant dans la chambre de plénum (4) et dans la chambre de manipulation (3) peut être réglé, de préférence modifié pendant l'état de fonctionnement de décontamination.

7. Système de décontamination selon l'une quelconque des revendications précédentes,
**caractérisé en ce que**
les moyens de distribution ultrafins (19) comprennent un évaporateur commun ou un atomiseur commun qui est relié à la conduite d'alimentation de plénum (25) et à la conduite d'alimentation de manipulateur (27) ou que les moyens de distribution ultrafins (19) ont des évaporateurs ou atomiseurs séparés destinés à alimenter la conduite d'alimentation de plénum (25) et la conduite d'alimentation de manipulateur (27).

8. Système de décontamination selon l'une quelconque des revendications précédentes,
**caractérisé en ce qu'**
un filtre (8) à haute performance pour particules en suspension, notamment un filtre ULPA ou un filtre HEPA, par lequel le flux d'air (9) produit par le ventilateur de circulation (7) peut être appliqué à la chambre de plénum (4) est disposé entre la chambre de plénum (4) et la chambre de production de circulation d'air (6), qui est disposé de préférence au-dessus de la chambre de plénum (4).

9. Système de décontamination selon l'une quelconque des revendications précédentes,
**caractérisé en ce qu'**
un catalyseur (10) destiné à dégrader du décontaminant (20) lors du mode de circulation d'air est assigné au ventilateur de circulation (7) qui peut être commandé à l'aide des moyens de commande.

10. Système de décontamination selon la revendication 9,
**caractérisé en ce que**
le catalyseur (10) est disposé de manière stationnaire par rapport au ventilateur de circulation (7) et les moyens de commande sont configurés pour commander le ventilateur de circulation (7) de telle manière que le ventilateur de circulation (7) est désactivé pendant l'état de fonctionnement de décontamination, ou qu'une dérivation peut être établie entre le catalyseur (10) et le ventilateur de circulation (7), de préférence en décalant le catalyseur (10) ou les moyens de conduction d'air assigné à celui-ci, et que les moyens de commande sont configurés pour commander le ventilateur de circulation (7) de telle manière qu'il est en marche pendant l'état de fonctionnement de décontamination quand la dérivation est établie.

11. Système de décontamination selon l'une quelconque des revendications précédentes,
**caractérisé en ce que**
l'au moins une conduite d'alimentation de plénum (25) et/ou l'au moins une conduite d'alimentation de manipulateur (27) débouchent respectivement dans la chambre de plénum (4) et dans la chambre de manipulation (3) par l'intermédiaire d'une buse (33), chaque buse (33) ayant plusieurs orifices de sortie (35), qui sont dirigés dans différentes directions et qui sont répartis de préférence sur un tronçon conique (36) et sur un tronçon cylindrique adjacent au tronçon conique (36).

12. Système de décontamination selon l'une quelconque des revendications précédentes,
**caractérisé en ce que**
les moyens de distribution ultrafins (19) sont reliés à une conduite d'air porteur (23) destiné à alimenter les moyens de distribution ultrafins (19) en air porteur de l'extérieur de la chambre de travail pendant la phase de décontamination, l'air porteur servant à absorber de la vapeur de décontaminant ou à atomiser du décontaminant.

13. Système de décontamination selon l'une quelconque des revendications précédentes,
**caractérisé en ce que**
l'au moins une conduite d'alimentation de manipulateur (27) débouche dans la chambre de manipulation (3) dans la région d'une paroi latérale de la chambre de manipulation et/ou qu'aucune unité de travail ou fonctionnelle pharmaceutique n'est disposée directement verticalement au-dessous de l'extrémité de la conduite d'alimentation de manipulateur.

14. Procédé de fonctionnement d'un système de décontamination (1), de préférence selon l'une quelconque des revendications précédentes, comprenant un isolateur (2) ayant une chambre de manipulation (3) et une chambre de plénum (4) qui est disposée au-dessus de la chambre de manipulation (3) et qui est alimentée en un flux d'air (9) à l'aide d'au moins un ventilateur de circulation (7) disposé dans une chambre de production de circulation d'air (6), une membrane (5) destinée à produire un flux d'air (11) laminaire, de préférence vertical, dans la chambre de manipulation (3) pendant un état de fonctionnement de travail étant disposée entre la chambre de plénum (4) et la chambre de manipulation (3), la chambre de manipulation (3) étant reliée à la chambre de production de circulation d'air (6) de manière à conduire de l'air par un canal d'air de retour (12) disposé entre deux plaques transparentes (13, 14), comprenant en outre des moyens de distribution ultrafins (19) destinés à produire un mélange décontaminant-air sur la base d'un réservoir de décontaminant liquide, les moyens de distribution ultrafins (19) étant reliés à la chambre de plénum (4) par au moins une conduite d'alimentation de plénum (25) qui débouche dans la chambre de plénum (4) et par laquelle la chambre de plénum (4) est alimentée directement en mélange décontaminant-air,
**caractérisé en ce que**
les moyens de distribution ultrafins (19) sont reliés directement à la chambre de manipulation (3) par au moins une conduite d'alimentation de manipulateur (27) par laquelle la chambre de manipulation (3) est alimentée directement en mélange décontaminant-air à l'état de fonctionnement de décontamination.

15. Procédé selon la revendication 14,
**caractérisé en ce que**
la chambre de plénum (4) et la chambre de manipulation (3) sont alimentées simultanément en mélange décontaminant-air et/ou que la chambre de plénum (4) et la chambre de manipulation (3) sont alimentées en mélange contaminant/air l'une après l'autre, notamment plusieurs fois en alternance, et/ou qu'un débit volumique des flux volumiques de mélange décontaminant-air coulant dans la chambre de plénum (4) et dans la chambre de manipulation (3) est réglé, de préférence modifié pendant l'état de fonctionnement de décontamination.

16. Procédé selon l'une quelconque des revendications 14 ou 15,
**caractérisé en ce qu'**
un passage à partir de l'au moins une conduite d'alimentation de plénum (25) à l'au moins une conduite d'alimentation de manipulateur (27) est fermé pendant l'état de fonctionnement de travail afin d'empêcher l'air coulant dans la chambre de manipulation (3) à partir de la chambre de plénum (4) par la conduite d'alimentation de plénum (25) et la conduite d'alimentation de manipulateur (27) quand le passage n'est pas fermé de perturber le flux d'air (11) laminaire dans la chambre de manipulation (3).
